# EUROPEAN PATENT APPLICATION

(11) **EP 3 091 007 A1**
(43) Date of publication of application: **09.11.2016**
(21) Application number: 16168398.2
(22) Date of filing: 04.05.2016
(51) Int. Cl.: C07D 401/06, C07D 211/70

(54) **PROCESS FOR THE PREPARATION OF PIPERIDINE COMPOUNDS**

(30) Priority: 08.05.2015 IT UB20150417
(71) Applicant: Dipharma Francis S.r.l., 20021 Baranzate (MI) (IT)
(72) Inventor: ATTOLINO, Emanuele, 20021 BARANZATE (MI) (IT); RIZZO, Elisabeth, 20021 BARANZATE (MI) (IT); ROSSI, Davide, 20021 BARANZATE (MI) (IT)
(74) Representative: Bianchetti Bracco Minoja S.r.l.

(57) **Abstract**

The present invention relates to a process for the preparation of intermediates useful in the synthesis of efinaconazole and their use in the synthesis of efinaconazole.

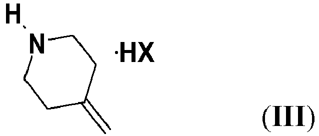

## Description

### FIELD OF INVENTION

The present invention relates to a process for the preparation of intermediates useful in the synthesis of efinaconazole and their use in the synthesis of efinaconazole.

### PRIOR ART

(2*R*,3*R*)-2-(2,4-difluorophenyl)-3-(4-methylenepiperidinyl)-1-(1H-1,2,4-triazolyl)butan-2-ol of formula (I), also known as efinaconazole, 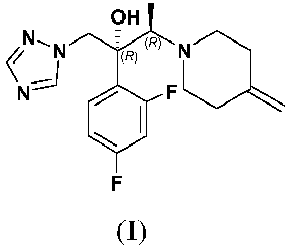 is an active pharmaceutical ingredient belonging to the class of triazole antifungals which is used in the topical treatment of distal and lateral subungual onychomycosis (DLSO), a chronic infection of the ungual apparatus mainly caused by dermatophytes and yeasts and characterised by discolouring, thickening and deformity of the nail.

The 10% topical solution of efinaconazole is marketed under the name of Jublia®.

Efinaconazole is known from EP0698606 B1, which describes its preparation by means of a process comprising opening of the optically pure epoxide of formula **(II)** with the 4-methylenepiperidine of formula **(IIIa),** according to Scheme 1 below.

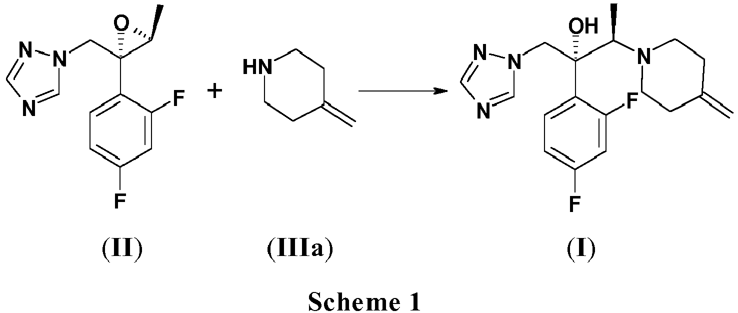

Said reaction is conducted in water or in a water/organic solvent mixture, wherein the organic solvent can be an ether, an alcohol or an amide, at reflux, in the presence of a large excess of 4-methylenepiperidine of formula **(IIIa),** namely 5 to 20 times the moles of the 4-methylenepiperidine of formula **(IIIa)** compared with the epoxide of formula **(II).** Under these conditions, the reaction leads to the formation of efinaconazole of formula **(I)** together with various by-products. Following the purification of the crude reaction product by chromatography or crystallisation, efinaconazole of formula **(I)** is isolated with a yield not exceeding 54%.

Patent US 8,871,942 subsequently described an improved process for the synthesis of efinaconazole **(I)** which involves opening the epoxide of formula **(II)** in the presence of a 4-methylenepiperidine salt of formula **(III),** such as the hydrobromide salt, and of an alkali or alkaline-earth metal hydroxide, in particular of lithium in an appropriate solvent, according to Scheme 2 below.

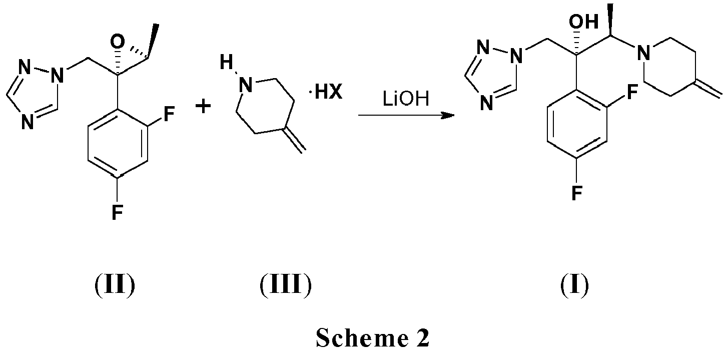

Said reaction proceeds more rapidly than the one described according to Scheme 1, but 4-methylenepiperidine free base of formula **(IIIa)** is still used to obtain the 4-methylenepiperidine salt of formula **(III).**

A 4-methylenepiperidine salt of formula **(III)** is obtained as reported in US 8,871,942 by treating 4-methylenepiperidine free base of formula **(IIIa)** with an organic or mineral acid in a solvent, as the skilled person is well aware.

WO97/11939 reports one of the most interesting syntheses of 4-methylenepiperidine free base of formula **(IIIa)** in terms of its preparation on an industrial scale, according to Scheme 3 below.

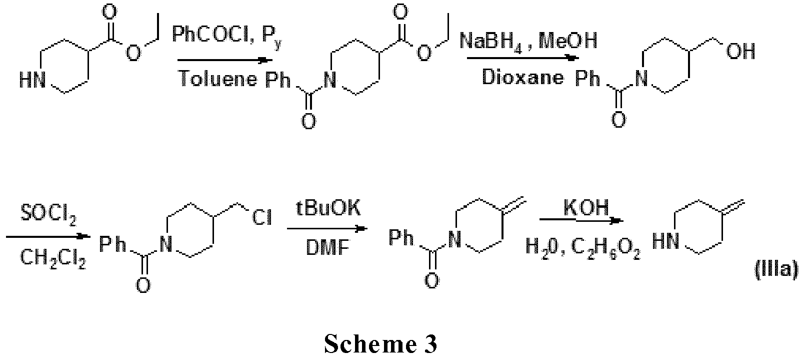

This process involves protecting the amine function of 4-ethyl isonipecotate as benzamide and reducing the resulting benzamide with NaBH₄ to obtain an alcohol which is first converted to chloride and then to olefin with tBuOK in DMF (dimethylformamide). Finally, the protected olefin is deprotected by treating it with KOH in an H₂O/glycol mixture at high temperature. The crude 4-methylenepiperidine of formula **(IIIa),** dissolved in the aqueous mixture, is then isolated by low-pressure distillation, and thus obtained as an oil with a water content ranging between 20% and 70% by weight. The 4-methylenepiperidine free base of formula **(IIIa)** thus obtained can then be converted into a 4-methylenepiperidine salt of formula **(III)** by methods known to the skilled person and as reported in US 8,871,942.

However, the drawback of the preparation process of 4-methylenepiperidine free base of formula **(IIIa)** described in WO97/11939, and therefore of the preparation process of a 4-methylenepiperidine salt of formula **(III)** as reported in US 8,871,942, on an industrial scale, lies mainly in the olefin deprotection step which leads to the formation of the 4-methylenepiperidine base of formula **(IIIa)**. In fact, the latter cannot be isolated from the reaction medium by simple extraction in organic solvent, but must necessarily be purified by means of laborious distillation at high temperature under vacuum.

The problem of the deprotection step has been partially solved in WO 2005/115398 which describes the preparation of intermediate 4-methylenepiperidine trifluoroacetate salt (intermediate 13B) by reacting a 4-methylenepiperidine derivative protected as tert-butylcarbamate (Boc, intermediate 13A) with trifluoroacetic acid in polar aprotic solvent CH₂Cl₂. Unfortunately Intermediate 13B revealed to be a colorless oil and as known in the art, any product obtained as an oil suffers of a low purity grade, and the impurities present in the oily material can jeopardize the purity degree of the final active substance. As a consequence intermediates obtained as an oil have to be submitted to cumbersome and time consuming purification processes.

There is therefore a need for an improved process for the preparation of a 4-methylenepiperidine salt of formula **(III)** in a solid state which eliminates all the disadvantages of the processes known to the prior art and can easily be used on an industrial scale for the synthesis of efinaconazole of formula **(I).** Said process should not involve the isolation of 4-methylenepiperidine of formula **(IIIa),** which is a water-soluble liquid that cannot be extracted in organic phase, cannot be isolated by simple evaporation of solvent, and is therefore difficult to purify. Said novel process should in particular involve the use of efficient, economical, operationally simple reaction conditions to obtain a 4-methylenepiperidine salt of formula **(III)** advantageously in a solid form, avoiding the use of toxic chlorinated solvents, in particular on an industrial scale and with high yields and purity.

### SUMMARY OF THE INVENTION

The invention provides a process for the preparation of efinaconazole of formula **(I)** 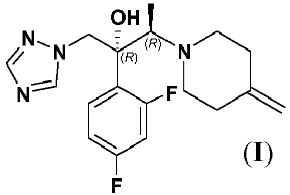 comprising reacting a compound of formula **(II)** 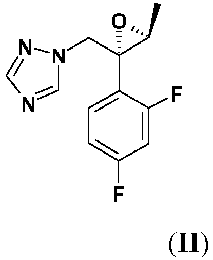 with a 4-methylenepiperidine salt of formula **(III)** in a solid state form 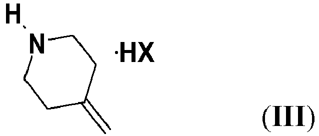 wherein HX is a sulfonic acid or a strong mineral acid, wherein 4-methylenepiperidine salt of formula **(III)** in a solid state form is obtained by a process comprising reacting a carbamate of formula **(IV)** 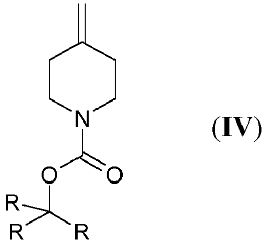 wherein each of substituents R, being the same or different, is H, a C₁-C₆ alkyl or aryl group; with an acid of formula

HX

as defined above, in a solvent.

### DETAILED DESCRIPTION OF THE INVENTION

The first subject of the present invention is a process for the preparation of a 4-methylenepiperidine salt of formula **(III)** in a solid state form 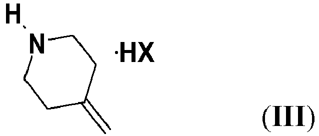 wherein HX is a sulfonic acid or a strong mineral acid, comprising reacting a carbamate of formula (IV) 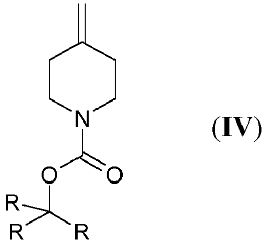 wherein each of substituents R, being the same or different, is H, a C₁-C₆ alkyl or aryl group; with an acid of formula

HX

as defined above, in a solvent.

A sulfonic acid is preferably selected from methanesulfonic, trifluoromethanesulfonic, p-toluenesulfonic and camphorsulfonic acid.

A strong mineral acid is preferably selected from the group comprising a hydrohalic acid, such as hydrobromic acid or hydrochloric acid; sulfuric acid; nitric acid; and phosphoric acid; preferably hydrobromic acid or hydrochloric acid, more preferably hydrochloric acid.

An acid HX is preferably a sulfonic acid, in particular p-toluenesulfonic acid.

A compound of formula **(III)** in a solid state form can be in amorphous or crystalline form, preferably in crystalline form.

A C₁-C₆ alkyl group, which can be straight or branched, is typically a C₁-C₄ alkyl group such as methyl, ethyl, propyl, isopropyl or butyl, isobutyl, tert-butyl, optionally substituted by one or more substituents, preferably by one to three substituents being the same or different, such as halogen, for example chlorine or fluorine, or aryl.

An aryl can be, for example, a phenyl or naphthyl group, typically phenyl. Said aryl group can be optionally substituted by one to three substituents selected independently from a straight or branched C₁-C₄ alkyl group, which in turn is optionally substituted by one to three atoms of halogen, typically fluorine; a hydroxy group; a C₁-C₄ alkoxy group, such as methoxy; a halogen atom, such as bromine or chlorine; a cyano group and a nitro group.

R is preferably a C₁-C₄ alkyl group, in particular methyl.

The reaction of a carbamate of formula **(IV)** with an acid of formula HX can be conducted in the presence of a polar protic or non-polar solvent, preferably a non-polar solvent.

A polar protic solvent can be selected, for example, from the group comprising a straight or branched C₁-C₆ alkanol, in particular methanol, ethanol, isopropanol or n-butanol; water; a weak C₁-C₄ alkyl carboxylic acid, such as acetic or propionic acid; and a mixture of two or more, preferably two or three, of said solvents; preferably methanol or isopropanol.

A non-polar solvent can be selected for example from the group comprising a hydrocarbon solvent, for example hexane, heptane, cyclohexane and toluene, and an ether, for example diethyl ether or methyltertbutylether, preferably toluene.

According to a preferred aspect of the invention, the reaction can be conducted in the presence of methanol, isopropanol or toluene, more preferably toluene.

Said reaction can be conducted at a temperature ranging between about 0°C and the reflux temperature of the solvent, preferably at the reflux temperature of the solvent.

The acid HX can be used in at least a stoichiometric quantity compared with the carbamate of formula **(IV).**

According to the process of the invention a 4-methylenepiperidine salt of formula **(III)** can be obtained directly in a solid state form, preferably in a crystalline form, from the end-of-reaction mixture, with no need to isolate the 4-methylenepiperidine free base of formula **(IIIa).**

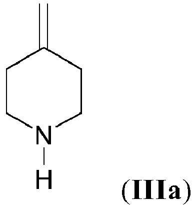

A 4-methylenepiperidine salt of formula **(III)** in a solid state form can be isolated by methods well known to the skilled person, such as filtration or centrifugation.

Surprisingly, a 4-methylenepiperidine salt of formula **(III)** in a solid state form as manufactured by the process of the invention is obtained with high yields and high chemical purity, typically higher than 98.5% as calculated by Gas Chromatography (GC).

The inventors of the present patent application have established that in the synthesis of a 4-methylenepiperidine salt of formula **(III)** by methods known in the art, for example by treating 4-methylenepiperidine free base of formula **(IIIa)** with a strong mineral acid, especially using a hydrohalic acid in a polar aprotic solvent, an impurity of formula **(A)** is often formed 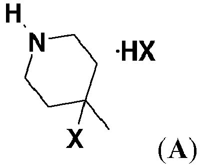 wherein substituent X is the anion of the mineral acid HX used in the reaction. Said impurity **(A)** is generated by Markovnikov addition of hydrohalic acid HX to the double bond of the 4-methylenepiperidine salt of formula **(III).**

However, wholly unexpectedly, a 4-methylenepiperidine salt of formula **(III)** in a solid state form prepared by the process according to the invention does not contain the impurity of formula **(A)**, and can be advantageously used to synthesize efinaconazole of formula **(I)** with highly purity degree.

A further subject of the invention is therefore a 4-methylenepiperidine salt of formula **(III)** with a strong mineral acid, as defined above, in particular a hydrohalic acid, in a solid state form, as obtained by process of the invention, with an impurity content of formula **(A)** below 0.1%, evaluated by HPLC Area% (A%).

A further subject of the invention is a process for the synthesis of efinaconazole **(I)** 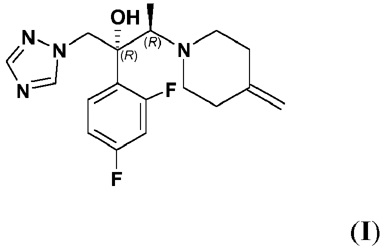 comprising reacting a compound of formula (II) 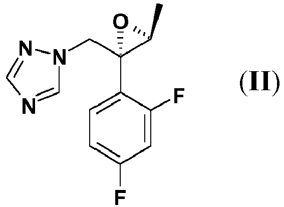 with a 4-methylenepiperidine salt of formula (III) in a solid state form, as obtained by the process of the invention 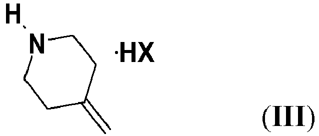 wherein HX is as defined above.

The reaction of a compound of formula **(II)** with a 4-methylenepiperidine salt of formula **(III)** can be carried out according to known methods, for example according to US 8,871,942 cited above.

According to a further aspect of the invention, efinaconazole of formula **(I),** obtained by the process of invention, when HX acid is a strong mineral acid, in particular a hydrohalic acid, has an impurity content of formula **(B)** 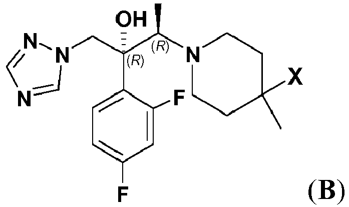 below 0.1% evaluated by HPLC Area % (A%), wherein X is the anion of the hydrohalic acid, as defined above.

Said impurity **(B)** is generated by opening of the epoxide of formula **(II)** 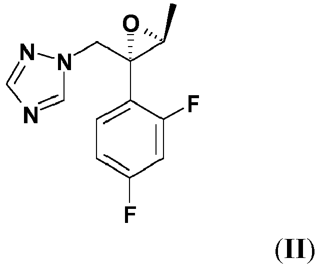 by reaction with the impurity of formula **(A).**

A further subject of the present invention is efinaconazole of formula **(I),** having an impurity content of formula (B) below 0.1% evaluated by HPLC Area % (A%), wherein X is the anion of an hydrohalic acid, as defined above.

A compound of formula **(IV)** can be prepared from a compound of formula **(V)** 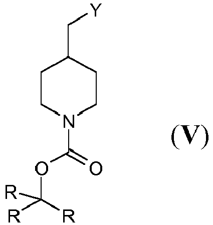 wherein substituents R are as defined above and Y is a halogen atom, preferably chlorine or bromine, or a leaving group, typically an RₐSO₃ group, wherein Rₐ is a C₁-C₆ alkyl group as previously defined, or an aryl group, as previously defined, or a heteroaryl group.

An aryl or heteroaryl can be a phenyl group or a 1-imidazolyl group respectively, typically phenyl.

Y is preferably an atom of chlorine or bromine, or as leaving RₐSO₃ group is preferably mesylate or tosylate. A compound of formula **(V)** can be converted to a compound of formula **(IV)** by treating a compound of formula **(V)** with a strong base in a solvent by well-known methods for elimination reactions. A strong base can be, for example, a strong organic base such as an alkali metal alkoxide, preferably potassium tert-butylate or sodium tert-butylate; or NaH; or a strong tertiary amine such as diazabicycloundecene (DBU).

A solvent can be, for example, a polar aprotic solvent, such as dimethylformamide (DMF) or dimethylacetamide (DMA), preferably DMF.

A further subject of the present invention is the use of a compound of formula **(IV)** as defined above, in the synthesis of efinaconazole of formula **(I).**

A compound of formula **(IV)** can be converted to another compound of formula **(IV)** by known methods; for example, a compound of formula **(IV)** wherein Y is a leaving group, preferably mesylate or tosylate, can be converted to a compound of formula **(IV)** wherein Y is an atom of chlorine or bromine, by methods well known to the skilled person, by treating a compound of formula **(IV),** wherein Y is a leaving group, with chloride or bromide ion donor reagents, for example with chlorides or bromides of alkylammonium quaternary salts, preferably with tetrabutylammonium bromide or chloride, more preferably with tetrabutylammonium chloride.

A compound of formula **(V)** can be prepared in turn from a compound of formula **(VI)** wherein each of R substituents is as defined above.

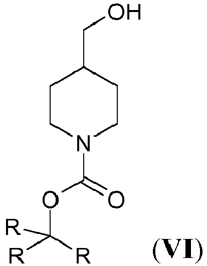

A compound of formula **(VI)** can typically be converted to a compound of formula **(V),** wherein Y is a halogen atom, by treating a compound of formula **(VI)** with hydrochloric acid in the presence of ZnCl₂ or hydrobromic acid, or with halogenating agents such as SOCl₂ or PCl₃.

A compound of formula **(V),** wherein Y is a leaving group, such as RₐSO₃-, can be obtained by activating the hydroxyl function of a compound of formula **(VI)** by treating it with a chloride of formula **(VII)** or an anhydride of formula **(VIII)** respectively,

RₐSO₂Cl **(VII)**

RₐSO₂OSO₂Rₐ **(VIII)**

wherein substituents Rₐ, which in a compound of formula **(VIII)** may be the same or different, are as defined above.

A compound of formula **(VI)** can preferably be converted to a compound of formula **(V),** wherein Y is CH₃SO₃-, by treating it with mesyl chloride by methods well-known to the skilled person.

A compound of formula **(VI)** can be prepared from a compound of formula **(IX),** wherein R₃ is H or a C₁-C₆ alkyl or aryl group as defined above, and each of R substituents is as defined above.

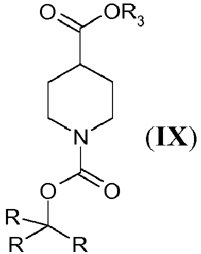

A compound of formula **(IX)** can be converted to a compound of formula **(VI)** by reducing a compound of formula **(IX)** with a hydride reducing agent, such as NaBH₄ or LiAlH₄ in a solvent, such as a cyclic ether. A compound of formula **(IX)** is preferably converted to a compound of formula **(VI)** using NaBH₄ in tetrahydrofuran (THF).

Finally, a compound of formula **(IX)** can be obtained by protecting the isonipecotate of formula **(X),** or a salt thereof, wherein substituent R₃ is as defined above. 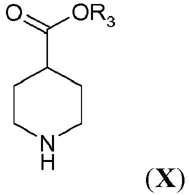 by treating it with a chloride of formula **(XI)** or an anhydride of formula **(XII)** respectively, 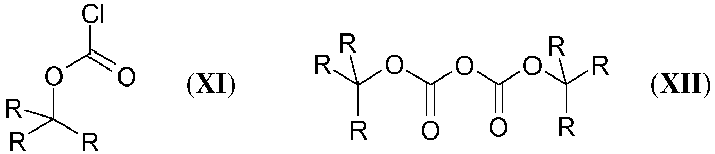 wherein each of substituents R, being the same or different, are as defined above, with amino-protecting methods well known to the skilled person.

The compounds of formulas **(X), (XI)** and **(XII)** are commercially available.

A salt of a compound of formula **(X)** is preferably a pharmaceutically acceptable salt thereof, such as the hydrochloride. A salt of a compound of formula **(X)** can be converted to the corresponding free compound by methods known to the prior art, such as release of the salt under basic conditions, for example by adding soda or potash.

The following examples further illustrate the invention.

### Example 1: Synthesis of ethyl N-(tert-butylcarbamyl)isonipecotate of formula (IX).

The 4-ethyl-isonipecotate of formula **(X)** (11.9 g, 76.0 mmol) is dissolved in AcOEt (60 ml) in a 4-necked 250 ml flask equipped with mechanical stirrer and thermometer and placed in a nitrogen atmosphere, and the resulting solution is treated with triethylamine (15.4 g, 152.0 mmol). The reaction mixture is cooled to 5°C and treated with a solution of di-tert-butyl dicarbonate Boc₂O (18.3 g, 84.0 mmol) in 10 minutes. At the end of dripping the reaction mixture is heated to 25°C and maintained under stirring. After 5 hours, GC-MS analysis demonstrates the disappearance of the starting compound. The end-of-reaction mixture is then diluted with H₂O (30 ml), and the phases obtained are separated. The aqueous phase is extracted with AcOEt and CH₂Cl₂, and the organic phase is washed sequentially with H₂O and a saturated aqueous solution of NaCl. The combined organic phases are dried on anhydrous Na₂SO₄, filtered and concentrated at low pressure to obtain the compound of formula **(IX)** as an oil (19.6 g). The product obtained is used as such, without further purifications in the next synthesis step.
¹H NMR (300 MHz, CDCl₃): 4.16-4.09 (q, 2H, J= 7.4 Hz), 4.03-3.99 (m, 2H), 2.82 (dt, 2H, J= 12.5 Hz, J= 3 Hz), 2.46-2.46 (m, 1H), 1.87-1.77 (m, 2H), 1.66-1.53 (m, 2H), 1.44 (s, 9H), 1.24 (t, 3H, J=7.4 Hz).
GC-MS: m/z = 200, 184, 156, 128, 112, 84, 57.

### Example 2: Synthesis of tert-butyl 4-hydroxymethylpiperidine-N-carboxylate of formula (VI).

The compound of formula **(IX),** obtained according to Example 1 (19.6 g, theoretical content 76.0 mmol) is dissolved in THF (65 ml) in a 250 ml flask equipped with mechanical stirrer, dropping funnel, condenser and thermometer, and solid NaBH₄ (5.8 g, 152.0 mmol) in portions is added to the resulting solution, maintaining the mixture under stirring and the temperature between 10°C and 15°C. The mixture is then heated to reflux and treated with MeOH (22.5 ml), added by slow dripping. The mixture is maintained under reflux, and after 3 hours the TLC and ¹H-NMR analyses demonstrate the total disappearance of the starting compound. The end-of-reaction mixture is then concentrated at low pressure. The residue obtained is diluted with H₂O cooled to 5°C and treated with 3M HCl to pH≈6. The resulting mixture is extracted with CH₂Cl₂. The combined organic phases are washed with a saturated aqueous solution of NaCl, dried on anhydrous Na₂SO₄, filtered and concentrated at low pressure, to obtain a compound of formula (VI) as oil (15.5 g). The product obtained is pure on NMR analysis and used as such, without further purifications in the next synthesis step.
¹H NMR (300 MHz, CDCl₃): 4.12 (brd, 2H, J=13.0 Hz), 3.49 (d, 2H, J=6.0 Hz), 2.69 (dt, 2H, J=13.0 Hz, J=2.1 Hz), 1.72-1.62 (m, 3H), 1.45 (s, 9H), 1.25-1.10 (m, 2H).

### Example 3: synthesis of tert-butyl 4-(methylsulphonyl-oxymethyl)piperidine-N-carboxylate of formula (V).

The compound of formula **(VI),** obtained according to Example 2 (15.5 g, 72.0 mmol), is dissolved in toluene (75 ml) in a 250 ml flask equipped with mechanical stirrer, dropping funnel and thermometer and placed in a nitrogen atmosphere. The resulting solution is treated with triethylamine (9.6 g, 95.0 mmol) and cooled to about 0°C. The solution is then treated with mesyl chloride (9.1 g, 75.7 mmol) added by slow dripping, maintaining the temperature below 5°C. After 40 minutes the end-of-reaction mixture is diluted with H₂O pre-cooled to 5°C, and the two phases are separated. The organic phase, washed with H₂O, dried on anhydrous Na₂SO₄, filtered and concentrated at low pressure, provides the compound of formula (V) as a solid (18.9 g), which is used without further purifications in the next step.
¹H NMR (300 MHz, CDCl₃): 4.16 (brd, 2H, J=12.6 Hz), 4.06 (d, 2H, J=6 Hz), 2.99 (s, 3H), 2.70 (brt, 2H, J=12.6 Hz), 1.72-1.67 (m, 3H), 1.44 (s, 9H), 1.25-1.17 (m, 2H). ¹³C-NMR (75 Hz, CDCl₃): 154.9, 79.8, 73.6, 43.5, 37.6, 36.3, 28.7, 28.5. GC-MS: m/z = 293 [M^{+.}], 281, 237, 220, 192, 158, 140, 126, 114, 96, 79, 57.
Melting point (DSC): 84°C.

### Example 4: synthesis of tert-butyl 4-chloromethylpiperidine-N-carboxylate of Formula (V).

The compound of formula **(V),** obtained according to Example 3 (18.9 g, 64.7 mmol), is dissolved in DMF (85 ml) in a 250 ml 3-necked flask equipped with magnetic stirrer, bubble condenser and thermometer and placed in a nitrogen atmosphere. The resulting solution is treated with tetrabutylammonium chloride (35.9 g, 129.1 mmol). The reaction mixture is heated to 55°C and maintained under stirring for 5 hours, then cooled to 20°C and treated with H₂O (100 ml) and methyl tert-butyl ether, MTBE (250 ml). The phases obtained are separated, and the aqueous phase is further extracted with MTBE (2×250 ml). The combined organic phases are washed with H₂O and a saturated aqueous solution of NaCl, dried on anhydrous Na₂SO₄, filtered and concentrated at low pressure, and supply the crude compound of formula (V) (12.2 g) as a solid, which is used without further purifications in the next step.
¹H NMR (300 MHz, CDCl₃): 4.13 (brd, 2H, J=13.3 Hz), 3.67 (d, 2H, J=6 Hz), 2.69 (dt, 2H, J=13.3 Hz, J=2.4 Hz), 1.82.1.73 (m, 3H), 1.46 (s, 9H), 1.25-1.12 (m, 2H).

### Example 5: synthesis of tert-butyl 4-methylenepiperidine-N-carboxylate of Formula (IV).

The compound of formula **(V),** obtained according to Example 4 (10.7 g, 46.1 mmol), is dissolved in DMF (43 ml) in a 250 ml 3-necked flask equipped with magnetic stirrer and thermometer and placed in a nitrogen atmosphere. The resulting solution is cooled to 10°C and treated with solid tBuOK (7.7g, 69.1 mmol) in portions. The mixture is heated to 25°C and maintained under stirring for 4 hours, then slowly poured onto a biphasic mixture of 1M HCl and toluene cooled to 5°C under stirring. The two phases are then separated, and the aqueous phase is further extracted with toluene. The combined organic phases are washed with H₂O, dried on anhydrous Na₂SO₄, filtered and concentrated at low pressure, to obtain the product of formula **(IV)** (9.0 g) as an oil, which is used without further purifications in the next step.
¹H NMR (300 MHz, CDCl₃): 4.73 (s, 2H), 3.41 (t, 4H, J=6.0 Hz), 2.19 (t, 4H, J=6.0 Hz), 1.46 (s, 9H). GC-MS: m/z = 197 [M^{+.}], 141, 124, 96, 82, 67, 57, 51.

### Example 6: synthesis of 4-methylenepiperidine hydrochloride of formula (III).

The compound of formula **(IV),** obtained according to Example 5 (3.0 g, 15.0 mmol), is dissolved in MeOH (18 ml) in a 50 ml 3-necked flask equipped with magnetic stirrer, bubble condenser and thermometer and placed in a nitrogen atmosphere. The resulting solution is treated with a 10.77% w/w solution of HCl in MeOH (5.7 g, 17.0 mmol), by adding it by slow dripping at 0°C, and then heated to 40°C. After 5 hours the reaction mixture is concentrated at low pressure to give the compound of formula **(III)** hydrochloride salt (1.9 g) as an oil. Said oil is taken up with toluene and dried by azeotropic distillation to provide a solid residue which is suspended in acetone and left to pulverize at 20°C for 1 hour, and then at 0°C for 2 hours. The solid is filtered through a Büchner funnel and stove-dried at 50°C to provide the compound of formula **(III)** hydrochloride salt (1.6 g), as a white solid with a yield of 78%. The impurity content of formula **(A)**, measured as HPLC Area % (A%), is less than 0.1%.
¹H-NMR (300 MHz, CDCl₃) 9.62 (brs, 2H), 4.86 (s, 2H), 3.15-3.23 (m, 4H), 2.57 (t, 4H, J= 6.3 Hz).

### Example 7: Synthesis of 4-methylenepiperidine hydrobromide (III).

The *tert*-butyl-4-methylenepiperidine-N-carboxylate of formula **(IV)** (190 g, 0.964 mol), obtained according to Example 5, is dissolved in 760 ml of isopropanol in a 2 L multi-necked flask equipped with mechanical stirrer, condenser and thermometer and placed in a nitrogen atmosphere. The resulting solution is heated to reflux and then treated with a 48% w/w aqueous solution of HBr (165.7 g, 0.983 mol) in about 20 minutes. After 16 hours the end-of-reaction mixture is concentrated at low pressure, and the residue obtained is taken up with isopropanol and dried by azeotropic distillation. Acetone (680 ml) is added to the resulting solid, and the suspension is maintained under stirring at 25°C for 30 minutes and then cooled to 0°C. After 2 hours the suspension is filtered through a Büchner funnel and washed with acetone, and the resulting solid is stove-dried at 50°C under vacuum to a constant weight to give 4-methylenepiperidine hydrobromide of formula **(III)** as a white solid (144.9 g) with a yield of 84%. The impurity content of formula **(A)**, measured as HPLC Area % (A%), is less than 0.1%.
Assay value ¹H-NMR 100.42%.
Melting point (DSC): 145°C.
¹H-NMR (300 MHz, CDCl₃) 9.41-9.13 (brs, 2H), 4.88 (s, 2H), 3.28-3.21 (m, 4H), 2.60 (brt, 4H, J=6 Hz). ¹³C-NMR (75 MHz, CDCl₃) 62.5, 41.7, 38.7, 34.7.

### Example 8: Synthesis of 4-methylenepiperidine p-toluenesulfonate (III).

In a 1000 ml reactor, equipped with a mechanical stirrer, thermometer and bubble condenser, in a nitrogen atmosphere p-toluensulfonic acid monohydrate (91.7 g, 482 mmol) is suspended in toluene (420 ml). The mixture is heated to 60°C and *tert*-butyl-4-methylenepiperidine-N-carboxylate of formula **(IV)** (90.6 g, 459 mmol) is slowly dropped therein. After 2 hours at 60°C the mixture is concentrated under reduced pressure and dried by azeotropic distillation to afford a solid. After cooling to 0°C, the solid is filtered on buchner and dried in oven at 50°C to yield the compound 4-methylenpiperidine paratoluensulfonate (96.5 g, 358 mmol) in a 78% yield, with chemical purity higher than 98.5% as measured by GC.
¹H-NMR (300 MHz, CDCl₃): 2.36 (s, 3H), 2.44 (t, 4H), 3.19 (m, 4H), 4.81 (s, 2H), 7.19 (d, 2H, J=7.8 Hz), 7.74 (d, 2H, J=8.1 Hz), 8.9 (bs, 2H).
¹³C-NMR (75 MHz, CDCl₃): 21.5, 31.1, 45.6, 111.9, 125.9, 129.2, 140.1, 140.7, 141.7. Melting point (DSC): 119 °C.

## Claims

1. A process for preparing a 4-methylenepiperidine salt of formula **(III)** in a solid state form 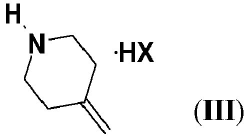 wherein HX is a sulfonic acid or a strong mineral acid, comprising reacting a carbamate of formula **(IV),** 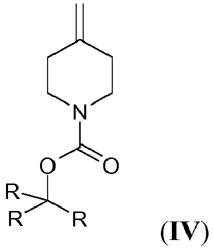 wherein each of the substituents R, being the same or different, is H, a C₁-C₆ alkyl or aryl group, with an acid of formula
HX
as defined above, in a solvent.

2. A process according to claim 1, wherein the acid is a sulfonic acid, preferably selected from the group comprising methanesulfonic acid, trifluoromethanesulfonic acid, p-toluenesulfonic acid and camphorsulfonic acid.

3. A process according to claim 2, wherein a sulfonic acid is preferably p-toluenesulfonic acid.

4. A process according to claim 1, wherein the acid is a strong mineral acid, preferably selected from the group comprising a hydrohalic acid, such as hydrobromic or hydrochloric acid; sulfuric acid; nitric acid and phosphoric acid, preferably hydrochloric acid.

5. A process according to claims 1-4, wherein the solvent is a polar protic or non-polar solvent, preferably a non-polar solvent.

6. A process according to claims 1-5, wherein the solvent is selected from methanol, isopropanol and toluene, preferably toluene.

7. A process according to claims 1-6, wherein the acid HX is used in at least a stoichiometric amount compared with the carbamate of formula **(IV).**

8. A process according to claims 1-6, wherein a 4-methylenepiperidine salt of formula **(III)** with a strong mineral acid, in particular a hydrohalic acid, in a solid state form, has a content of impurity of formula **(A)** 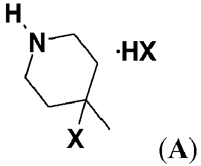 lower than 0.1% evaluated by A% HPLC, wherein HX is a hydrohalic acid and X is the anion of the hydrohalic acid HX.

9. A process according to claims 1-7, further comprising manufacturing efinaconazole of formula **(I)** 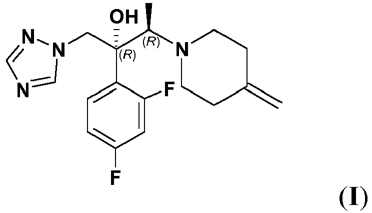 by a process comprising reacting a compound of formula **(II)** 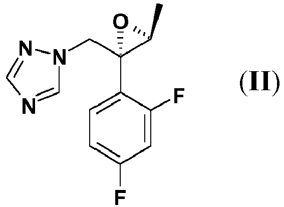 with a 4-methylenepiperidine salt of formula **(III)** in a solid state form,

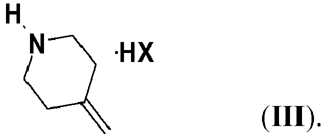

10. A process according to claim 9, wherein, when HX acid is a strong mineral acid, in paticular a hydrohalic acid, efinaconazole of formula (I) has a content of impurity of formula **(B)** 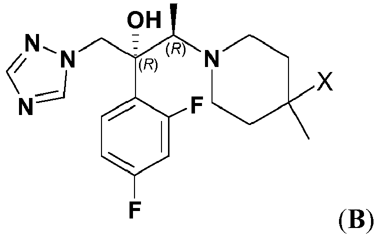 lower than 0.1% evaluated by A% HPLC, wherein X is the anion of the hydrohalic acid HX.

11. A process according to claims 1-7 and 9, wherein a compound of formula **(IV)** is prepared starting from a compound of formula **(V)** 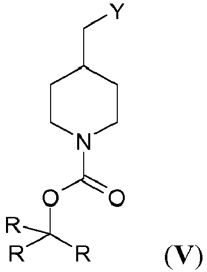 wherein R is as defined in claim 1 and Y is a halogen atom, preferably chlorine or bromine, or a leaving group, typically an RₐSO₃ group, wherein Rₐ is a C₁-C₆ alkyl, aryl, or heteroaryl group.

12. A 4-methylenepiperidine salt of formula **(III)** in a solid state form, as defined in claim 1, having a content of impurity of formula **(A)** 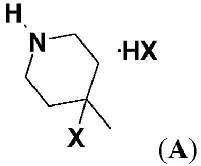 lower than 0.1% evaluated by A% HPLC, wherein HX is a hydrohalic acid, as defined in claim 4, and X is the anion of the hydrohalic acid HX.

13. Efinaconazole of formula **(I)** having a content of impurity of formula **(B)** 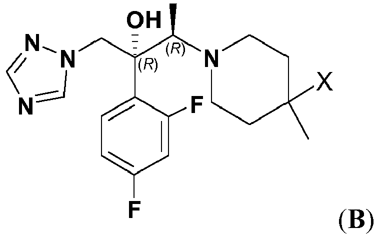 lower than 0.1% evaluated by A% HPLC, wherein X is the anion of the hydrohalic acid HX, as defined in claim 4.

14. Use of a compound of formula **(IV),** as defined in claim 1, in the manufacture of efinaconazole of formula (I)

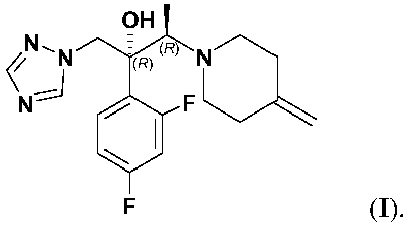

15. Use of a compound of formula **(V),** as defined in claim 11, in the manufacture of efinaconazole of formula **(I).**
